# EUROPEAN PATENT APPLICATION

(11) **EP 4 738 648 A1**
(43) Date of publication of application: **06.05.2026**
(21) Application number: 24210254.9
(22) Date of filing: 31.10.2024
(51) Int. Cl.: H02J 50/10, A61B 5/055, H02J 50/40, H02J 50/60, H02J 50/70, H02J 50/80

(54) **METHOD AND SYSTEM FOR WIRELESS CHARGING OF ONE OR MORE DETACHABLE ACCESSORY DEVICES DURING ACQUISITION OF MAGNET RESONANCE IMAGING DATA**

(71) Applicant: Koninklijke Philips N.V., 5656 AG Eindhoven (NL)
(72) Inventor: WEIß, Steffen, Eindhoven (NL)
(74) Representative: Philips Intellectual Property & Standards

(57) **Abstract**

The invention concerns a method for wireless charging of one or more detachable accessory devices during acquisition of magnet resonance imaging data. The method comprising the following steps: Receiving magnet resonance system data from an MR system; Receiving charging data from a charger of a detachable accessory device comprising at least a charging field of the charger; Determining an influence of the charging field of the charger on magnet resonance imaging data; Charging at least one detachable accessory device during acquisition of magnet resonance imaging data, and reducing the influence of the charging field on the magnet resonance imaging data during charging of the at least one detachable accessory device by using the magnet resonance system data. The invention further concerns an apparatus for wireless charging of one or more detachable accessory devices during acquisition of magnet resonance imaging data.

## Description

### FIELD OF THE INVENTION

The invention relates to the field of charging in the vicinity of magnetic fields, in particular to the field of wireless charging of detachable accessory devices.

More specifically, the invention relates to the field of wireless charging of detachable accessory devices near magnetic resonance imaging systems.

### BACKGROUND OF THE INVENTION

A magnetic resonance system may be equipped with one or more different detachable accessory devices (DAD). On the other hand, these detachable accessory devices may be stored near the magnetic resonance system so that they are close by and ready for use. These DAD such as for example, MR coils, user pads as mobile screens and input devices, an ECG detection unit, a respiratory sensor should be stored near or at and moved with the magnetic resonance system. For example, these devices should be moved with a portable magnetic resonance system. A DAD is fully sealed and wirelessly chargeable for allowing simple cleaning and avoiding battery exchange in hospital and/or patient environments. Therefore, wireless charging should be done at a storage place of the DAD. Such a DAD should also be small and lightweight for a good workflow and good mobility of the DAD and the entire magnetic resonance system. This results in the technical need for a low-capacity battery and to recharge as soon as the DAD is idle. A standard wireless charging interferes with magnetic resonance imaging due to radio frequency interference and a modulation of the magnetic field. Accordingly, immediate charging of the DAD may be impossible due to current imaging.

### SUMMARY OF THE INVENTION

Therefore, there exists a need for optimizing wireless charging of detachable accessory devices (DAD), in particular for optimizing wireless charging during an acquisition of a magnetic resonance imaging.

An object of the invention is to provide an effective and improved charging of detachable accessory devices during an acquisition of a magnetic resonance imaging without impairing the received imaging data of the magnetic resonance system. In particular, it is the object of the invention to reduce, or even avoid any influence of a charger of one or more detachable accessory devices for reducing, or even avoiding imaging artefacts.

The object of the present invention is solved with the subject matter of the independent claims, wherein further embodiments are incorporated in the dependent claims.

According to a first aspect of the invention, a method for wireless charging of one or more detachable accessory devices during acquisition of magnet resonance imaging data is provided. The method comprises the steps of receiving magnet resonance system data from an MR system; receiving charging data from a charger of a detachable accessory device comprising at least a charging field of the charger; determining an influence of the charging field of the charger on the magnet resonance imaging data; charging at least one detachable accessory device during acquisition of magnet resonance imaging data, and reducing, or even avoiding the influence of the charging field on the magnet resonance imaging data during charging of the at least one detachable accessory device by using the magnet resonance system data.

In the context of the present invention, the term "detachable accessory device" may be understood to describe any portable device used during an image acquisition of the MR system. These detachable accessory devices (DAD) can be removed from the MR system or can be removed from a location near the MR system to be used. For example, a detachable accessory device may be MR coils, user pads as mobile screens and input devices, an ECG detection unit, and a respiratory sensor, but the examples are not limited to this list. Any other detachable accessory device, which needs to be used during an image acquisition of an MR System.

In the context of the present invention, the term "MR system" should be understood to describe any MR system, in particular the invention may be applicable for portable MR imaging devices. Portable MR systems can be moved within a hospital and in other environments. These portable MR systems may be able to provide more accessible MR imaging and do not require a large and fixed hospital installation including an RF (radio frequency) shielded room and technical room. These systems can be wheeled around and plugged into normal mains power used at the point of care, for example at the bed of the patient. Further, they can be used in emergency departments, intensive care units and operating rooms. Even if the invention may be applicable for portable MR systems, the invention is not limited to the portable systems. An MR system, such as fixed or portable systems, comprise MRI hardware components, such as RF coils. These RF coils have at least two functions, firstly, to excite magnetization by broadcasting the RF power (Tx-Coil) and secondly, to receive the signal from the patient (Rx-Coil) that is used to reconstruct the MR image. Typically, this signal is order of magnitudes lower that RF emitted by electrical equipment, such as for example charger for DAD. To provide good image quality standard MR systems within a fixed hospital installation are located in an RF-screened room which is a Faraday cage that prevents any RF noise from entering the room. But when DAD's are used in the room their RF noise cannot be prevented inside the room. Further, portable MR systems are subject to any RF noise produced by neighbored equipment, and they are not shielded against external-magnetic field perturbation which may therefore impair image quality. Therefore, with the inventions as described herein both standard and portable MR systems are able to acquire MR imaging data while neighbored equipment, such as DAD's can be charged.

In the context of the present invention the term "magnet resonance imaging data" shall be understood to describe data received by the MR system from a subject being scanned in an MR imaging scan, wherein the magnet resonance imaging data is then used by the MR system to reconstruct MT images of the subject.

In the context of the present invention the term "magnet resonance system data" shall be understood to describe data that defines the magnetic resonance imaging scan and MR image reconstruction, including, but not limited to, information about a magnetic field of the MR system, magnetic field strength, receive band of the MR system, a sample sequence and its timing information and further.

In the context of the present invention the term "charging data" shall be understood to describe data derived from the charger, or the charging system. It may describe information about a type of the charger, the charging frequency, an electromagnetic charging field, a charging duration, and a charging capacity and further.

In other words, the method according to the first aspect provides steps for at least reducing, or even avoiding or eliminating the influence of a charger for a DAD on the received MR image data from which the images are reconstructed. The charging step may be performed in a wireless charging manner, which simplifies cleaning and avoids batter exchange. As there is a need for recharging as soon as possible, the user of the charger and/or the MR system may put the DAD at its charging location at the MR system and it should immediately start charging to ensure maximum charge at a next use. Accordingly, the method provides charging and imaging at the same time. Due to the analysis of the magnetic resonance system data and the charging data and the determination of the resulting influence of the charging field onto the magnetic resonance image data, the described method allows that even for a portable MR system with a low main magnetic field the detrimental influence of wireless charging on the magnetic resonance image data and the resulting MR images is reduced . In other words, due to the knowledge about MR system data, an influence or any deviations on resulting MR imaging data can be determined. Accordingly with the method described herein an immediate charging of the DAD becomes possible during MR image acquisition.

The charging of the DAD is performed wirelessly. Wireless charging uses the qi standard based on inductive coupling at radio frequencies. Low power devices can be delivered up to 15 W, and medium power devices up to 75 W. A start charging frequency may be 140 kHz, but due to variation of receiver load and coupling conditions between transmitter and receiver an actual frequency may be adapted in a range from 105 kHz to 205 Hz, for example. From MR systems their field frequency and operating frequency may be known, such that this information can be provided for the method.

The methods described herein may be computer implemented methods comprising elements for carrying out the different steps. For example, the method may be implemented in a computer and may be carried out using a computer system, having storage mediums for storing the received magnetic resonance system data and/or the charging data, and a processor which may be configured for controlling steps of the method and configured for controlling the charging of the DAD during the image acquisition.

It should be noted that any feature, function and/or element described in the following with reference to the apparatus for wireless charging equally applies to the method, and vice versa. Accordingly, any feature, function, step and/or element described in the following with reference to one aspect of the present disclosure equally applies to any other aspect of the present disclosure.

According to an exemplary embodiment of the invention, the influence of the charging field may be a harmonic of the charging field of the charger, in particular it may be the n-th harmonic of the charging field. The magnet resonance system data may comprise at least an MR receive band of the MR system; and wherein the step of determining the influence may comprise determining an influence of the charging field of the charger on magnet resonance imaging data in the MR receive band.

According to an exemplary embodiment of the invention, the method further may comprise the step, after determining the influence of the charging field on the magnet resonance imaging data, identifying a forbidden transmit band in the charging field of the charger. Further, the step of reducing the influence of the charging field of the charger may comprise avoiding the forbidden transmit band by the charger by modifying a charging field frequency.

The forbidden transmit band may be similar to the MR receive band, such that the charger knows his transmit band, can further determine whether this transmit band would be in the receive band. Or in other words, which parts of the transmit band would be in the receive band of the MR system. With this information the charger is able to determine the part of the transmit band which should be avoided, hence the forbidden transmit band. Differently speaking, the forbidden transmit band may comprise all frequencies which would have harmonic waves in the MR receive band. These frequencies should be avoided, hence not be used during charging a DAD. For instance, the frequencies may lie in the kHz range. By avoiding the forbidden transmit band any RF interference of the charger into the MR reception are avoided. It should be noted that when describing that the forbidden transmit band should be avoided, it is meant that the influence of the forbidden transmit band should be reduced. Ideally the forbidden transmit band may be avoided, such that no frequencies fall into the forbidden transmit band of the charger. In reality it may be a reduction of the forbidden transmit band, which not completely eliminates all frequencies but almost all of the influence thereof.

According to an exemplary embodiment of the invention, the step of avoiding the forbidden transmit band may further comprise the steps of determining the set of harmonics of the charging field and shifting the charging field frequency and thereby shifting the set of harmonics out of the MR receive band.

The set of harmonics may comprise endless harmonics, but only the set up to the MR receive band is of importance, whereas all higher harmonics may have no influence on the MR receive band. For example, for a portable MR system a low magnetic field around 64 mT is used corresponding to an MR receive band at about 2,7 MHz with a typical width of the receive band of 70 kHz. With the above-described exemplary charging frequency in a range of 105 to 205 kHz, the n-th harmonic of the charging field, with for example n= 20, may fall into the MR receive band and may cause strong image artefacts. A shift of the charging frequency of Δf may lead to a shift of n*Δf of the n-th harmonic. Therefore, by only a minor shifting the charging frequency of less than 70kHz/2n = 1.75kHz it may be possible to shift the n-th harmonic out of the MR receive band. Such a small shift may not impair the efficacy of wireless charging notably. The exact required shift may depend on an imaging sequence, a position and size of an imaging field-of-view in the MR readout direction, which are key MR parameters and determine the MR receive band for that imaging sequence. It should be noted that it may be possible to keep the MR receive band free from any harmonics because their spacing (between 105kHz and 205kHz) is larger than the width of the MR receive band.

According to an exemplary embodiment of the invention, the magnet resonance system data may may further comprise at least a magnetic field B0 of the MR system, wherein the influence of the charging field may further be a spatially and temporal dependent offset of the magnetic field B0 of the MR system.

These further parameters may be received alternatively or additionally to the parameter of the MR receive band. Hence, the influence of the charging field may be a harmonic of the charging field (frequency) of the charger and alternatively or additionally the spatially and temporal dependent offset of the magnetic field B0 of the MR system. Therefore, there may be one specific influence which should be reduced (one of the two as described) or a combination of both influences may need to be reduced.

According to an exemplary embodiment of the invention, the method may further comprise the step of determining a spatial dependence and a temporal dependence of the magnetic field B0 of the MR system. Further, the charging data from the charger of the detachable accessory device may comprise at least a position and orientation of the charger with respect to the MR system. Additionally, the method may comprise the step of determining a deviating magnetic field B0 of the MR system influenced by the charger, and compensating the determined deviation magnetic field B0 in a signal demodulation or in reconstruction of the magnetic resonance imaging data.

The charger itself may be positioned at different locations, the information about these different locations may be used for the charging data. Practically it may be usual that the charger comprises a fixed position at the MR system itself, hence its position is constant.

The deviating magnetic field B0 of the MR system caused by the charger may be the so called off-resonance. The compensating may be described as a signal demodulation defined by the off-resonance term or as any form of consideration of the off-resonance term in reconstruction. In the context of the present invention the term "off-resonance" shall be understood to describe a deviation of the main magnetic field of an MR system from its value without presence of the charging field, which may lead to image artifacts. The main magnetic field may be understood to describe the magnetic field B0 of the MR system. The off-resonance may lead to signal corruption resulting in MR imaging artefacts. By determining the influence of the off-resonance, hence the spatially and temporal dependence of the magnetic field B0 of the MR system, and compensating this influence these image artefacts can be reduced, or even avoided.

The spatially and temporal dependent offset may be described as ΔB(x,t) of the B0 main magnetic field of a, for example portable MR system. Both the spatial and temporal dependence may be calculated by applying the Biot-Savart-law to the geometry and input current of the transmit antenna of the charger, which are part of the charging data. The method step of compensating described herein, may compensate the respective off-resonance Δω(x,t) = γ* ΔB0(x,t) in image reconstruction. Alternatively, this term may be used as correction during demodulation of the raw image signal received from the MR system.

According to an exemplary embodiment of the invention, the wireless charging of the at least one detachable accessory device may be performed simultaneously with the acquisition of the magnetic resonance imaging data. In particular, wireless charging can be performed, when the MR imaging is performed. In detail, the charging can be performed when the MR system receives the MR imaging data, wherein with the present invention artefacts caused by charging can be reduced, even avoided.

According to an exemplary embodiment of the invention, the method may further comprise, when charging more than one detachable accessory device, determining for each respective detachable accessory device the influence of the charging field. In other words, each respective DAD may have a respective influence during charging, wherein this respective influence may be determined. Additionally, the method may comprise the step of reducing for each detachable accessory device the influence of the charging field during charging. According to this embodiment, the different embodiments, hence method steps, described for charging at least one DAD and for using at least one charger, may be applicable in a similar manner for a plurality of DAD's. For example, for each DAD attached for charging on the charger (or a base of the charger) the influence of the charging field for charging each respective DAD may be determined and avoided by applying the method steps as described herein (for each respective DAD).

When using one charger for a plurality of detachable accessory devices, for example when different bases in the single charger for each DAD are used, for each base, hence for each DAD, the respective charging field must be determined and the influence of each charging field from each different base can be determined by the method.

This means, it may be possible to use one charger for a plurality of DAD's, in particular a plurality of bases or so-called dockets for the DAD's. Each base may have a different charging field, which may cause different influences on the magnetic resonance imaging data.

According to an exemplary embodiment of the invention, the method may further comprise receiving charging data from a plurality of chargers. Additionally, the method may further comprise the step or steps of determining an influence of each charging field of each of the plurality of chargers on the MR receive band, charging at least one detachable accessory device in the respective one of the plurality of chargers during magnet resonance imaging, and reducing the influence of each charging field during charging of the at least one detachable accessory device.

Differently speaking, when using more than one charger for each charger the charging field is determined, or received from the respective charger, and the influence from each charging field of each respective charger at the MR receive band is determined with the present method. Hence, the method steps are also applicable to a plurality of chargers and their respective influence on the magnetic resonance imaging data, which can be reduced/avoided in a similar, or even equal, manner as for one single charger. Consequently, the number of chargers, hence the number of DAD attached or attachable to an MR system is not limited.

According to a second aspect of the present invention a method for wireless charging of one or more detachable accessory devices during acquisition of magnet resonance imaging data is provided. The method according to the second aspect comprises the following steps: receiving magnet resonance system data from an MR system comprising at least an MR signal reception period and an MR signal non-reception period of an MR sequence of the MR system; determining one or more non-reception periods in the MR sequence; charging the one or more detachable accessory device only during non-reception periods of the MR sequence.

This method may be an alternative for the method as described with the first aspect according to the present invention. The method according to the second aspect of the present invention, may use a time-interleaved charging field with the MR image acquisition allowing uncompromised MR imaging data.

MR sequences are organized such that MR signal reception acquisition may take place in only a fraction of the imaging time. Reception and non-reception sequences alternate with repetition times of for example 5 ms to 100 ms and a fraction of 10 % to 30 % spent on reception. The charging is interleaved with the MR imaging such that the charging takes place only during the non-reception sequences of the imaging sequence.

The input data may be the same as in the method according to the first aspect of the present invention, hence the magnetic resonance system data, and the charging data, wherein the influence of the charging field is reduced and/or avoided by controlling the charging time in such a way that only during non-reception times in the MR sequence is performed.

According to a third aspect of the present invention an apparatus for wireless charging of one or more detachable accessory devices during acquisition of magnet resonance imaging data is provided. The apparatus comprises a processor configured to receive magnet resonance system data from an MR system; receive charging data from a charger of a detachable accessory device comprising at least a charging field of the charger; determine an influence of the charging field of the charger on the magnet resonance imaging data; and control the charger by providing the information about the influence of the charging field to the charger and to control the charger to reduce the influence of the charging field on the magnet resonance imaging data during charging of the at least one detachable accessory device during acquisition of magnet resonance imaging data by using the magnet resonance system data.

The apparatus may be an external device or system with which any MR system may be equipped, or which may be attached in the vicinity of a fixed MR system. Further, the apparatus may comprise additional components for storing, such as storage, the received information, for receiving, such as receiving unit, information from the MR system and the charger, and for transmitting, such as a transmitting unit, the received information or the processed information to other devices, such as the charger, an external computer, or the MR system. This list of components may not be limited, other elements may be added depending on the application of the apparatus.

The herein described apparatus may be configured to perform the method according to the first and the third aspect of the present invention with the different embodiments as described herein.

According to an exemplary embodiment of the invention, the apparatus may be the MR system, wherein the charger may be attached at the MR system or in the vicinity of the MR System.

In other words, the MR system itself may be able to perform the method as described with the different embodiments herein. Further, it may be possible that the apparatus may be a unit of the MR system, such that the apparatus is an integral part of the MR system. Further, it may also be possible that the apparatus may be the charger itself or the charger may be equipped with the apparatus. Furthermore, it may be possible to use a system comprising the above-described apparatus, an MR System and a charger for detachable accessory devices.

For example, if the apparatus is a charger, the processor may be a part of the charger. If the apparatus is the MR System, the processor may be an integral part of the MR system such that the MR system is able to control the processor which in turn controls the charger. Other data paths may also be possible between the charger, the MR System and the processor as long as the herein described method for wirelessly charging detachable accessory devices during medical resonance imaging can be applied.

According to an exemplary embodiment of the invention, the processor may be further configured to control one or more chargers which may be able to charge one or more detachable accessory devices. The processor may be further configured to, for each charger and/or for each detachable accessory device, to determine a respective influence of the charging field on the magnet resonance imaging data in MR receive band and/or on the magnetic field B0 of the MR system.

According to an exemplary embodiment of the invention, the charger may be a sinus charger providing a sinus-wave output signal.

In other words, the charger provides a sinus-wave charging signal in an ideal case. In reality, this signal may be subject of small deviations from an ideal sinus-wave, and it is transmitted only for a limited time period for charging, which both result in a set of harmonics, but each harmonic having a narrow frequency band compared to the MR receive band. Therefore, the narrow frequency band of the charger and its harmonics can be easily shifted out of the broad MR receive band.

According to a fourth aspect of the present invention, a computer program comprising instructions, when the program is executed by a computer, cause the computer to carry out the method as described herein with exemplary embodiments. In detail, the program may cause the computer to carry out the method for wireless charging of one or more detachable accessory devices during acquisition of magnet resonance imaging data according to the first aspect and/or according to the second aspect of the present invention as described with any one of the embodiments herein.

The computer program element may be part of a computer program, but it can also be an entire program by itself. For example, the computer program element may be used to update an already existing computer program to get to the present invention.

The program element may be stored on a computer readable medium. The computer readable medium may be seen as a storage medium, such as for example, a USB stick, a CD, a DVD, a data storage device, a hard disk, or any other medium on which a program element as described above can be stored.

For example, the method and/or the computer program element may be implemented as part of the operating system of the device. In that case, the vendor of the device provides the above functions to charge the detachable accessory device during acquisition of magnetic resonance imaging data as part of the device as delivered to all customers. Alternatively, the vendor may provide a switch in the system settings of the device where the user may have the option to switch off or customize the function. For instance, the method and/or the computer program element may be implemented as an App, for instance as part of the MR System in a hospital or as part of a portable MR system.

According to a further aspect of the invention, a computer-readable medium comprising instructions which, when executed by a computer, cause the computer to carry out the method of any one of the embodiments as described herein. In detail, the computer-readable medium may cause the computer to carry out the method for wireless charging of one or more detachable accessory devices during acquisition of magnet resonance imaging data according to the first aspect and/or according to the second aspect of the present invention as described with any one of the embodiments herein.

It has to be noted that embodiments of the invention have been described with reference to different subject matters. In particular, some embodiments have been described with reference to device/apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the device type claims and features of the method type claims is considered as to be disclosed with this application.

It should be noted that the term "comprising" does not exclude other elements or steps and "a" or "an" does not exclude a plurality. Also, elements described in association with different embodiments may be combined. It should also be noted that reference signs in the claims should not be construed as limiting the scope of the claims.

### BRIEF DESCRIPTION OF THE DRAWINGS

The aspects defined above, and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 illustrates schematically an MR system and an apparatus for wireless charging a detachable accessory device according to an exemplary embodiment of the invention.
Fig. 2 illustrates schematically an MR system including an apparatus for wireless charging a detachable accessory device according to an exemplary embodiment of the invention.
Fig. 3 illustrates a flow diagram showing schematically a method for charging a detachable accessory device according to a first exemplary embodiment of the invention.
Fig. 4 illustrates a flow diagram showing further steps of a method for charging a detachable accessory device according to the first exemplary embodiment of the invention.
Fig. 5 illustrates a flow diagram showing schematically a method for charging a detachable accessory device according to another exemplary embodiment of the invention.

### DETAILED DESCRIPTION OF EMBODIMENTS

The illustrations in the drawings are schematic. It is noted that in different figures similar or identical elements are provided with the same reference signs.

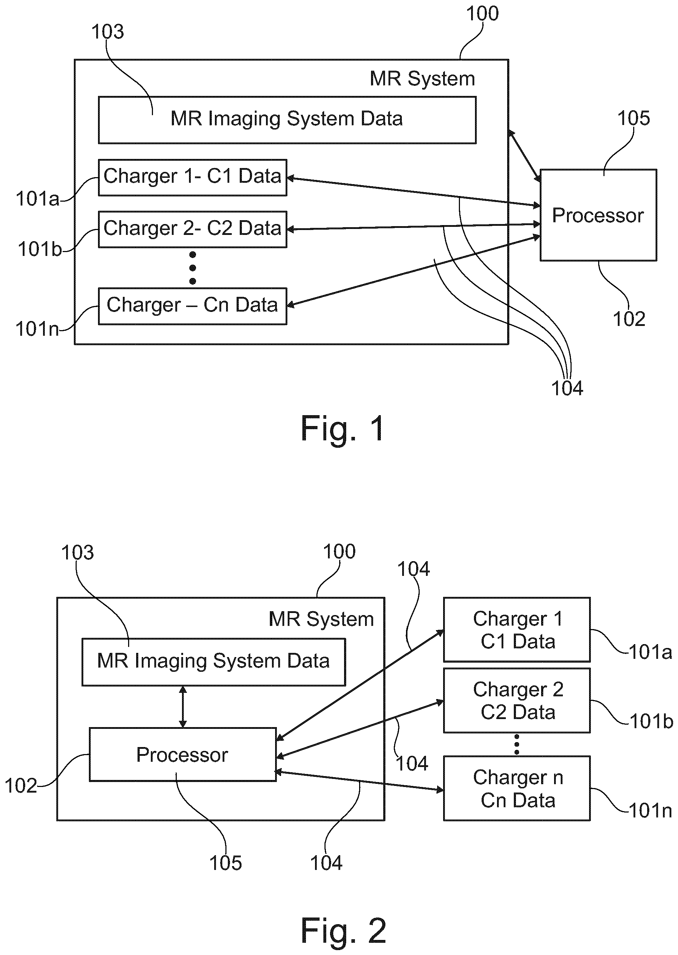

Fig. 1 illustrates schematically an MR system 100 and an apparatus 102 for wireless charging a detachable accessory device according to an exemplary embodiment of the invention. The apparatus 102 comprises a processor 105 configured to receive magnet resonance system data 103 from the MR system 100; receive charging data 104 (C1 Data to Cn Data) from a charger 101 (a to n) of a detachable accessory device comprising at least a charging field of the charger 101; determine an influence of the charging field of the charger 101 on magnet resonance imaging data 103; and control the charger 101 by providing the information about the influence of the charging field to the charger 101 and to control the charger 101 to reduce the influence of the charging field on the magnet resonance imaging data 103 during charging of the at least one detachable accessory device during acquisition of magnet resonance imaging data 103. In Fig. 1 a plurality of chargers 101 (a to n) is illustrated; the use of only one charger 101 may also be possible. Further, the apparatus 102 in Fig. 1 is illustrated as an external device of the MR system 100, and the charger(s) 101 are attached to the MR system 101. The DAD's which can be attached, and connected for charging, to the respective charger 101 are omitted in Fig. 1. It may be clear that for each charger at least one DAD may be attached for being charged, or a plurality of DAD's may be attached to the first charger 101a, the second charger 10b and to the further charger 101n. It may also be possible that different charging combination may be used, such as the first charger 101a is configured to charge a plurality of DAD, s by having respective bases for the DAD and the second charger 102b may be configured to charge at least one DAD, or vice versa.

Fig. 2 illustrates schematically an MR system 100 including an apparatus 102 for wireless charging a detachable accessory device according to an exemplary embodiment of the invention. The components as illustrated with Fig. 2 are similar to the components described with Fig. 1. The difference between these Figures is that in Fig. 2 the apparatus 102 is illustrated as a part of the MR system 100 and the chargers 101 (a to n) are illustrated as external parts of the MR system 100. The functionality of the apparatus in Fig. 2 is the same as described with Fig. 1. The same applies to the configuration of the chargers 101 (a to n). It may also be possible that the chargers 101 (a to n) and the apparatus 102 are parts of the MR system 101.

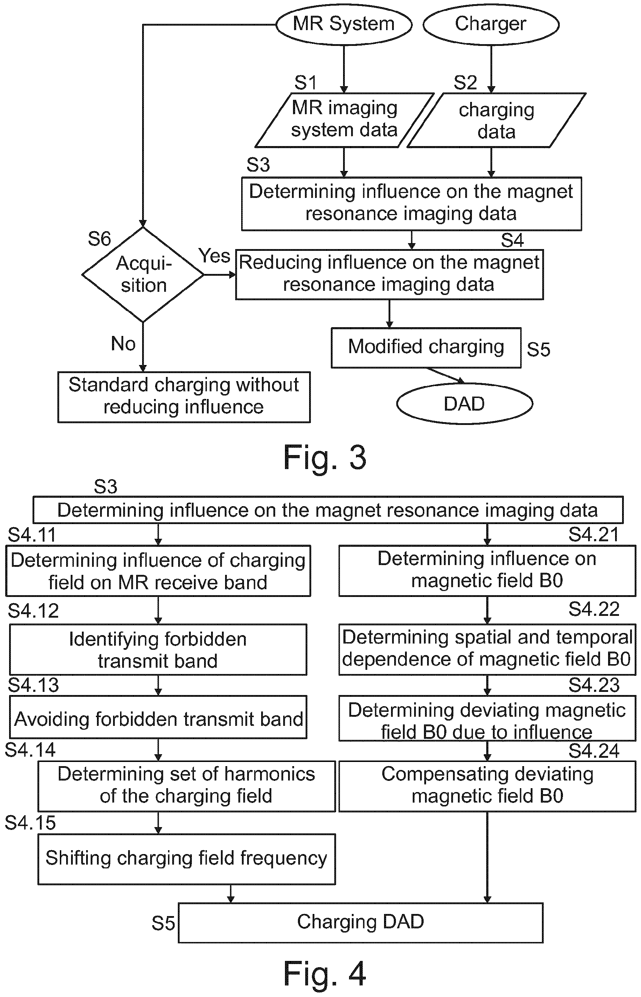

Fig. 3 illustrates a flow diagram showing schematically a method for charging a detachable accessory device according to a first exemplary embodiment of the invention. The order of the various steps shown in Fig. 3 and in the following Figures referring to a method and their steps is provided for the sake of illustration and may be altered without departing from the scope of the present teachings. In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein. The apparatus as illustrated with Fig. 1 and Fig. 2 may be a non-limiting example of such a hardware computer system.

The method illustrated in Fig. 3 is a method for wireless charging of one or more detachable accessory devices during acquisition of magnet resonance imaging data is provided. The method comprises the steps of S1 receiving magnet resonance system data from an MR system 100; step S2 receiving charging data from a charger 101 of a detachable accessory device comprising at least a charging field of the charger; step S3 determining an influence of the charging field of the charger 101 on magnet resonance imaging data; step S5 charging at least one detachable accessory device during step S6 acquisition of magnet resonance imaging data, and step S4 reducing, or even avoiding the influence of the charging field on the magnet resonance imaging data during step S5 charging of the at least one detachable accessory device. Step S4 may e.g. be the calculation of a new charging frequency for which the MR receive band is avoided. S5 is then the corresponding charging. The steps S4 and S5 may be connected in such a manner that after performing the charging S5 the influence may be reduced by step S4, which may be the case for the reconstruction embodiment es described herein. Contrary both steps S4 and S5 may be performed in a simultaneous or semi-simultaneous manner, which may be the case for demodulation embodiment as described herein. This means when the DAD is charged the influence is reduced/avoided, or an instruction may be provided before charging on how to reduce the influence and during charging the reducing of the influence is maintained or surveilled. Step S6 of the acquisition of the MR image can be performed during the same time period as the charging. Fig. 3 illustrates that when no acquisition is performed during charging, then any reduction of the influence is not necessary. Accordingly, only when a DAD is charged during the acquisition step S6 a reduction of the influence of the charging field on the magnetic resonance imaging data is necessary and will be performed.

Fig. 4 illustrates a flow diagram showing further steps of a method for charging a detachable accessory device according to the first exemplary embodiment of the invention. In Fig. 4 more details and intermediate steps between the step S3 of determining the influence and the step S5 of charging the DAD(s) is shown. In particular, sub-steps of step S4 reducing the influence on the magnet resonance imaging data are illustrated with steps S4.11 to S4.15 and steps S4.21 to S4.24. The method may differentiate between different influences on the magnet resonance imaging data. If the influence on the magnet resonance imaging data, hence the influence of the charging field is a harmonic of the charging field of the charger 101, in particular the n-th harmonic of the charging field; and wherein the magnet resonance system data 103 comprises at least an MR receive band of the MR system 100, the method as illustrated with Fig. 3 may further comprise the steps of step S4.11 determining the influence of the charging field of the charger on magnet resonance imaging data in the MR receive band. After step S4.11 determining the influence of the charging field on the magnet resonance imaging data, step S 4.12 is performed which is identifying a forbidden transmit band in the charging field of the charger, and wherein the step S4 of reducing the influence of the charging field of the charger 101 comprises the step S 4.13 avoiding the forbidden transmit band by the charger 101 by modifying a charging field frequency. Further, the step S4.13 of avoiding the forbidden transmit band further comprises the steps S4.14 of determining the set of harmonics of the charging field, and step 4.15 of shifting the charging field frequency and thereby shifting the set of harmonics out of the MR receive band. Afterwards, the step S5 of charging may be performed.

Moreover, if the magnet resonance system data 103 further comprises at least a magnetic field B0 of the MR system 100 and wherein the influence of the charging field is further a spatially and temporal dependent offset of the magnetic field B0 of the MR system 100. The method may perform the steps 4.21 to S4.24 additionally or alternatively to the steps of S4.11 to S4.15. The steps S4.21 to S 4.24 comprise the step S4.21 of determining a spatial dependence and a temporal dependence of the magnetic field B0 of the MR system 100, wherein the charging data 104 from the charger 101 of the detachable accessory device further comprises at least a position and orientation of the charger with respect to the MR system; afterwards step S4.23 can be performed which is determining a deviating magnetic field B0 of the MR system influenced by the charger. The next step is S4.24 compensating the determined deviation magnetic field B0 in a signal de-modulation or in reconstruction of the magnetic resonance imaging data. After the compensation is performed, the charger is controlled to charge the DAD in step S5.

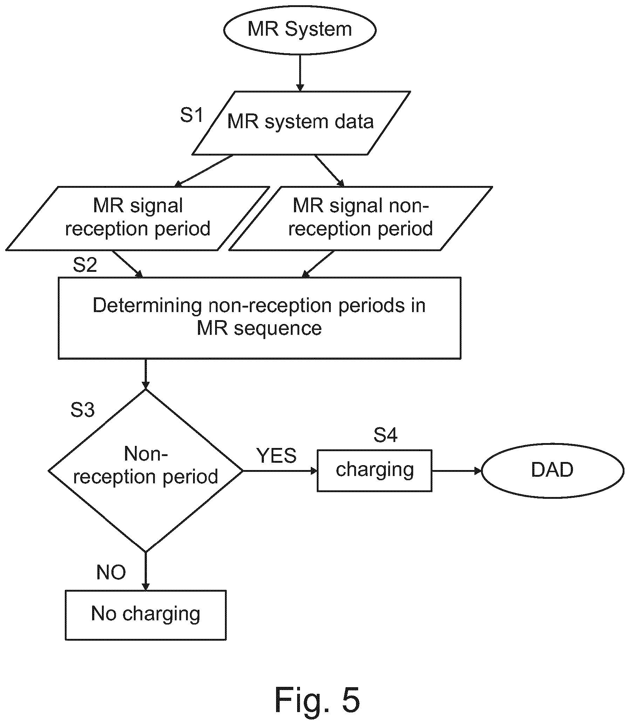

Fig. 5 illustrates a flow diagram showing schematically a method for charging a detachable accessory device according to another exemplary embodiment according to the second aspect of the invention. The method comprises the following steps: step S1 receiving magnet resonance system data 103 from an MR system 100 comprising at least an MR signal reception period and an MR signal non-reception period of an MR sequence of the MR system 100. Afterwards step S2 is performed, which is determining one or more non-reception periods in the MR sequence. During the determination of whether a non-reception period or a reception period is present the method may additionally comprise the step of a selection in S3. This may be used as a security measure for avoiding charging during reception periods. Only if a non-reception period is determined the charging in step S4 for the DAD is allowed and performed.

### LIST OF REFERENCE SIGNS:

100 MR System
101 charger
102 apparatus
103 magnetic resonance system data
104 charging data
105 processor
S1 to S5 method steps

## Claims

1. A method for wireless charging of one or more detachable accessory devices during acquisition of magnet resonance imaging data, wherein the method comprises the following steps:
Receiving magnet resonance system data from an MR system;
Receiving charging data from a charger of a detachable accessory device comprising at least a charging field of the charger;
Determining an influence of the charging field of the charger on the magnet resonance imaging data;
Charging at least one detachable accessory device during acquisition of magnet resonance imaging data, and
Reducing the influence of the charging field on the magnet resonance imaging data during charging of at least one detachable accessory device by using the magnet resonance system data.

2. The method according to claim 1,
wherein the influence of the charging field is a harmonic of the charging field of the charger, in particular the n-th harmonic of the charging field;
wherein the magnet resonance system data comprises at least an MR receive band of the MR system;
wherein the step of determining the influence comprises determining an influence of the charging field of the charger on magnet resonance imaging data in the MR receive band.

3. The method according to claim 1 or 2, wherein the method further comprises
after determining the influence of the charging field on the magnet resonance imaging data, identifying a forbidden transmit band in the charging field of the charger, and
wherein the step of reducing the influence of the charging field of the charger comprises avoiding the forbidden transmit band by the charger by modifying a charging field frequency.

4. The method according to claim 3, the step of avoiding the forbidden transmit band further comprises the steps of
Determining the set of harmonics of the charging field,
Shifting the charging field frequency and thereby shifting the set of harmonics out of the MR receive band.

5. The method according to any one of the preceding claims,
wherein the magnet resonance system data further comprises at least a magnetic field B0 of the MR system;
wherein the influence of the charging field is further a spatially and temporal dependent offset of the magnetic field B0 of the MR system.

6. The method according to claim 5, further comprising the steps of
Determining a spatial dependence and a temporal dependence of the magnetic field B0 of the MR system;
wherein the charging data from the charger of the detachable accessory device further comprises at least a position and orientation of the charger with respect to the MR system;
Determining a deviating magnetic field B0 of the MR system influenced by the charger,
Compensating the determined deviation magnetic field B0 in a signal de-modulation or in reconstruction of the magnetic resonance imaging data.

7. The method according to any one of the preceding claims,
wherein the wireless charging of the at least one detachable accessory device is performed simultaneously with the acquisition of the magnetic resonance imaging data.

8. The method according to any of the preceding claims,
wherein the method further comprises, when charging more than one detachable accessory device, determining for each respective detachable accessory device the influence of the charging field; and
reducing for each detachable accessory device the influence of the charging field during charging.

9. The method according to any of the preceding claims,
wherein the method further comprises receiving charging data from a plurality of chargers;
determining an influence of each charging field of each of the plurality of chargers on the MR receive band;
Charging at least one detachable accessory device in the respective one of the plurality of chargers during magnet resonance imaging, and
Reducing the influence of each charging field during charging of the at least one detachable accessory device.

10. A method for wireless charging of one or more detachable accessory devices during acquisition of magnet resonance imaging data, wherein the method comprises the following steps:
Receiving magnet resonance system data from an MR system comprising at least an MR signal reception period and an MR signal non-reception period of an MR sequence of the MR system;
Determining one or more non-reception periods in the MR sequence;
Charging the one or more detachable accessory device only during non-reception periods of the MR sequence.

11. An apparatus for wireless charging of one or more detachable accessory devices during acquisition of magnet resonance imaging data, comprising
a processor configured to
receive magnet resonance system data from an MR system;
receive charging data from a charger of a detachable accessory device comprising at least a charging field of the charger;
determine an influence of the charging field of the charger on the magnet resonance imaging data;
control the charger by providing the information about the influence of the charging field to the charger and to control the charger to reduce the influence of the charging field on the magnet resonance imaging data during charging of the at least one detachable accessory device during acquisition of magnet resonance imaging data by using the magnet resonance system data.

12. The apparatus according to claim 11, wherein
the apparatus is the MR system, wherein the charger is attached to the MR system or in the vicinity of the MR System.

13. The apparatus according to claim 11 or 12,
wherein the processor is configured to control one or more charger which is able to charge one or more detachable accessory devices,
wherein the processor is further configured to, for each charger and/or for each detachable accessory device, to determine a respective influence of the charging field on the magnet resonance imaging data in MR receive band.

14. The apparatus according to any of claims 11 to 13,
wherein the charger is a sinus charger providing a sinus-wave output signal.

15. A computer program comprising instructions, which, when the program is executed by a computer, cause the computer to carry out the method according to any one of the claims 1 to 10.
